(19) Europäisches Patentamt
European Patent Office
Office européen des brevets

(11) EP 1 097 139 B1

(12) EUROPEAN PATENT SPECIFICATION

(45) Date of publication and mention of the grant of the patent:
**04.12.2002 Bulletin 2002/49**

(21) Application number: **99935261.0**

(22) Date of filing: **14.07.1999**

(51) Int Cl.[7]: **C07D 215/56**, C07D 215/36, C07D 215/22, A61K 31/47

(86) International application number:
**PCT/SE99/01271**

(87) International publication number:
**WO 00/003992 (27.01.2000 Gazette 2000/04)**

(54) **QUINOLINE DERIVATIVES**
CHINOLINDERIVATE
DERIVES DE QUINOLINE

(84) Designated Contracting States:
**AT BE CH CY DE DK ES FI FR GB GR IE IT LI LU MC NL PT SE**
Designated Extension States:
**AL LT LV MK RO SI**

(30) Priority: **15.07.1998 SE 9802550**

(43) Date of publication of application:
**09.05.2001 Bulletin 2001/19**

(73) Proprietor: **Active Biotech AB**
**220 07 Lund (SE)**

(72) Inventors:
- **BJÖRK, Anders**
  **S-237 36 Bjärred (SE)**
- **JÖNSSON, Stig**
  **S-222 41 Lund (SE)**
- **FEX, Tomas**
  **S-226 52 Lund (SE)**
- **HEDLUND, Gunnar**
  **S-224 56 Lund (SE)**

(74) Representative: **Bergvall-Eftring, Stina Lena et al**
**Dr. Ludwig Brann Patentbyra AB**
**P.O. Box 17192**
**104 62 Stockholm (SE)**

(56) References cited:

| | |
|---|---|
| **EP-A1- 0 059 698** | **WO-A1-92/18483** |
| **WO-A1-93/06829** | **WO-A1-93/19756** |
| **WO-A1-95/24195** | **WO-A1-95/24196** |
| **WO-A1-95/24395** | **GB-A- 2 290 786** |

- **PATENT ABSTRACTS OF JAPAN & JP 07 224 040 A (FUJITSAWA PHARMACEUT CO LTD) 22 August 1995**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 097 139 B1

Printed by Jouve, 75001 PARIS (FR)

**Description**

FIELD OF THE INVENTION

The present invention relates to novel quinoline derivatives with a thio-substitutent incorporated into the 5-position, to methods for their preparation and to compositions containing them. The novel quinoline derivatives are appropriate for clinical treatment of diseases resulting from autoimmunity, such as multiple sclerosis, insulin-dependent diabetes mellitus, systemic lupus erythematosus, rheumatoid arthritis, inflammatory bowel disease and psoriasis and, furthermore, for diseases where pathologic inflammation plays a major role, such as asthma, atherosclerosis, stroke and Alzheimer's disease. More particularly, the present invention relates to novel quinoline derivatives suitable for the treatment of, for example, multiple sclerosis and its manifestations.

BACKGROUND OF THE INVENTION

Autoimmune diseases, e.g., multiple sclerosis (MS), insulin-dependent diabetes mellitus (IDDM), systemic lupus erythematosus (SLE), rheumatoid arthritis (RA), inflammatory bowel disease (IBD) and psoriasis represent assaults by the body's immune system which may be systemic in nature, or else directed at individual organs in the body. They appear to be diseases in which the immune system makes mistakes and, instead of mediating protective functions, becomes the aggressor (1).

MS is the most common acquired neurologic disease of young adults in western Europe and North America. It accounts for more disability and financial loss, both in lost income and in medical care, than any other neurologic disease of this age group. There are approximately 250.000 cases of MS in the United States.

Although the cause of MS is unknown, advances in brain imaging, immunology, and molecular biology have increased researchers' understanding of this disease. Several therapies are currently being used to treat MS, but no single treatment has demonstrated dramatic treatment efficacy. Current treatment of MS falls into three categories: treatment of acute exacerbations, modulation of progressive disease, and therapy for specific symptoms.

MS affects the central nervous system and involves a demyelination process, i.e., the myelin sheaths are lost whereas the axons are preserved. Myelin provides the isolating material that enables rapid nerve impulse conduction. Evidently, in demyelination, this property is lost. Although the pathogenic mechanisms responsible for MS are not understood, several lines of evidence indicate that demyelination has an immunopathologic basis. The pathologic lesions, the plaques, are characterised by infiltration of immunologically active cells such as macrophages and activated T cells (2).

In WO 92/18483 quinoline derivatives substituted with a $R_AS$ $(O)_n$-group ($R_A$ = lower alkyl or aryl; n = 0 - 2) are claimed, stated to possess an immunomodulating, anti-inflammatory and anti-cancer effect. However, said substituents are only disclosed in the 6-position of the quinoline ring.

Further, in US Pat. No. 4,547,511 and in US Pat. No. 4,738,971 and in EP 59,698 some derivatives of N-aryl-1,2-dihydro-4-substituted-1-alkyl-2-oxo-quinoline-3-carboxamide are claimed as enhancers of cell-mediated immunity. The compound

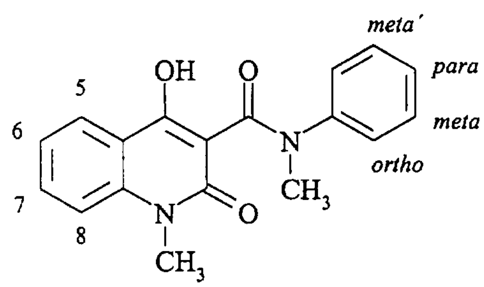

Roquinimex

known as roquinimex (Merck Index 12th Ed., No. 8418) belongs to this series of compounds. Roquinimex has been reported to have multiple immunomodulatory activities not accompanied with general immunosuppression.

Furthermore, in US Pat. No. 5,580,882, US Pat. No. 5,594,005, WO 95/24195 and WO95/24196 quinoline-3-carboxamide derivatives are claimed to be useful in the treatment of conditions associated with MS, type I diabetes,

inflammatory bowel disease and psoriasis, respectively. The particular preferred compound is roquinimex.

The substitution, i.e, type and pattern, of the above compounds, specifically mentioned in the prior art, places them outside the scope of the present invention.

In clinical trials comparing roquinimex to placebo, roquinimex was reported to hold promise in the treatment of conditions associated with MS (3, 4). There are, however, some serious drawbacks connected to some quinoline-3-carboxamide derivatives. For example, it has been found that roquinimex is teratogenic in the rat, and induces dose-limiting side effects in man, e.g., a flu-like syndrome, which prevents from using the full clinical potential of the compound.

DESCRIPTION OF THE INVENTION

A primary objective of the present invention is to provide structurally novel quinoline compounds which by virtue of their pharmacological profile, with high potency in experimental models and low level of side-effects, are considered to be of value in the treatment of disease resulting from autoimmunity and pathologic inflammation. Examples of such diseases are multiple sclerosis, insulin-dependent diabetes mellitus, systemic lupus erythematosus, rheumatoid arthritis, inflammatory bowel disease and psoriasis and other diseases where inflammation plays a major role, such as asthma, atherosclerosis, stroke and Alzheimer's disease. More particularly, the present invention relates to novel quinoline derivatives suitable for the treatment of, for example, multiple sclerosis and its manifestations.

The term "treatment" as used herein includes prophylaxis as well as relieving the symptoms of disease.

It has now surprisingly been found that the new and novel compounds of general formula (I)

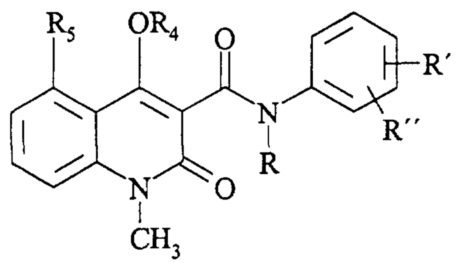

(I)

wherein

R is selected from Me, Et, n-Pr, iso-Pr, n-Bu, iso-Bu, sec.-Bu and allyl;

R' is selected from hydrogen, Me, MeO, fluoro, chloro, bromo, $CF_3$, and $OCH_xF_y$;

R" is selected from hydrogen and fluoro, with the proviso that R" is fluoro when R' is fluoro and further provided that when R' and R" are both hydrogen, R is not Me;

$R_4$ is selected from hydrogen and pharmaceutically acceptable inorganic cations, such as sodium, potassium and calcium, and organic cations such as monoethanolamine, diethanolamine, dimethylaminoethanol, morpholine and the like;

$R_5$ is selected from MeS, EtS, n-PrS, iso-PrS, MeSO, EtSO, $MeSO_2$ and $EtSO_2$;

wherein

$$x = 0 - 2,$$

$$y = 1 - 3$$

with the proviso that

$$x + y = 3;$$

and

Me is methyl, Et is ethyl, Pr is propyl and Bu is butyl;

are unexpectedly effective and specific in the treatment of individuals suffering from autoimmune and inflammatory

diseases.

The compounds of general formula (I) may exist in different tautomeric forms and all such forms where such forms exist are included herein. Also optical isomers and racemates of the compunds of general formula (I) where such forms exist are included herein.

In a preferred embodiment of the invention $R_4$ is selected from hydrogen and sodium,
$R_5$ is selected from MeS, and EtS,
R is selected from methyl, ethyl and propyl,
R' is selected from methoxy, fluoro, chloro and trifluoromethyl when R" is hydrogen, and R is methyl;
R" is selected from *meta'*- and *para*-fluoro when R' is *ortho*-fluoro and R is methyl; and
R'and R" are both hydrogen when R is ethyl and propyl.

Among the most preferred compounds of general formula (I) according to the present invention are:

N-ethyl-N-phenyl-1,2-dihydro-4-hydroxy-5-methylthio-1-methyl-2-oxo-quinoline-3-carboxamide,
N-n-propyl-N-phenyl-1,2-dihydro-4-hydroxy-5-methylthio-1-methyl-2-oxo-quinoline-3-carboxamide,
N-methyl-N-(4-methoxy-phenyl)-1,2-dihydro-4-hydroxy-5-methylthio-1-methyl-2-oxo-quinoline-3-carboxamide,
N-methyl-N-(4-chloro-phenyl)-1,2-dihydro-4-hydroxy-5-methylthio-1-methyl-2-oxo-quinoline-3-carboxamide,
N-methyl-N-(4-trifluoromethyl-phenyl)-1,2-dihydro-4-hydroxy-5-methylthio-1-methyl-2-oxo-quinoline-3-carboxamide,
N-methyl-N-(2,4-difluoro-phenyl)-1,2-dihydro-4-hydroxy-5-methylthio-1-methyl-2-oxo-quinoline-3-carboxamide,
N-methyl-N-(2,5-difluoro-phenyl)-1,2-dihydro-4-hydroxy-5-methylthio-1-methyl-2-oxo-quinoline-3-carboxamide,
N-ethyl-N-phenyl-1,2-dihydro-4-hydroxy-5-methylsulphinyl-1-methyl-2-oxo-quinoline-3-carboxamide,
N-n-propyl-N-phenyl-1,2-dihydro-4-hydroxy-5-methylsulphinyl-1-methyl-2-oxo-quinoline-3-carboxamide, and
N-methyl-N-(2,4-difluoro-phenyl)-1,2-dihydro-4-hydroxy-5- methylsulphinyl-methyl-2-oxo-quinoline-3-carboxamide.

Several spontaneously occurring autoimmune diseases in man have experimental models that are spontaneously occurring in certain strains of laboratory animals or can be induced in laboratory animals by immunisation with specific antigen(s) from the target organ.

Experimental autoimmune encephalomyelitis (EAE) as a model for autoimmune inflammatory diseases of the central nervous system (CNS) has been the most widely used model for the human disease multiple sclerosis.

Autoimmunity to type II collagen can experimentally be induced in certain strains of mice or rats and may lead to the development of polyarthritis. The collagen induced arthritis has several features in common with the human disorder rheumatoid arthritis.

The hallmark of asthma in humans is an increased reactivity of the airways to a range of chemical and physical stimuli. It is now widely accepted that products released from inflammatory cells, e.g., activated eosinophils, compromise epithelial integrity and promote bronchial hyperresponsiveness. The murine model of ovalbumin (OA)-induced lung inflammation is dominated by the temporally regulated influx of lymphocytes and eosinophils into the bronchial lumen.

Roquinimex has been found to induce the Beagle Pain Syndrome (BPS) (5, 6) in different breeds of beagle dogs. The disease is reflected by clinical and laboratory manifestations justifying BPS as a model for the flu-like syndrome induced by roquinimex in man.

The compounds of general formula (I) were assayed for inhibition of acute experimental autoimmune encephalomyelitis (aEAE) in mice. Roquinimex was used as treatment control and showed a more than 50% inhibition at ≥5 mg/kg. Surprising and unexpected results were obtained when introducing proper substitution in the 5-position of the quinoline ring. In comparison with roquinimex, the potency of the 5-methylthio substituted compound was increased a 100-fold. Corresponding substitution in the 6-position resulted in less active compounds, as shown in the examples. Furthermore, proper aromatic substitution of the 3-carboxamide moiety of the compounds of general formula (I) significantly reduced or even abolished the side-effects, i.e., the teratogenic effects and the BPS, of roquinimex. Thus, physicochemical properties of the 5-substituent in the quinoline moiety and the *ortho-, meta-* and/or, in particular, the *para-* substituent in the 3-carboxamide moiety are of major importance for an improved risk/benefit ratio in comparison with roquinimex. Also replacement of the methyl group on the carboxamide nitrogen with a higher alkyl group significantly reduced the side effects. Hence, the compounds of formula (I) have surprisingly been found to be both chemically and pharmacologically different from those drugs hitherto suggested for the treatment of MS and its manifestations.

All embodiments of the invention as disclosed in the claims are herewith included in the specification.

The following examples are intended to illustrate the invention without restricting the scope thereof.

The compounds of general formula (I) may be prepared by methods known in the literature and the following methods:

Method A:

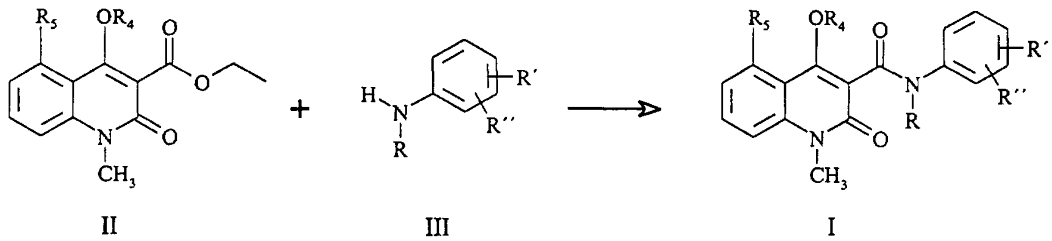

The compounds of general formula (I) may be prepared by known methods and, for example, as shown above, by reaction of an ester derivative of the quinoline carboxylic acid with an aniline in a suitable solvent such as toluene, xylene and the like. General methods for preparation of the quinoline carboxylic acid ester derivatives of formula (II) are described below. N-alkylated anilines of formula (III) are commercially available or known from literature, e.g., in Johnstone et al, J. Chem. Soc. 1969, 2223-2224. Compounds falling within the scope of formula (III) may be prepared by methods, which are generally analogous to those of said literature.

Method B:

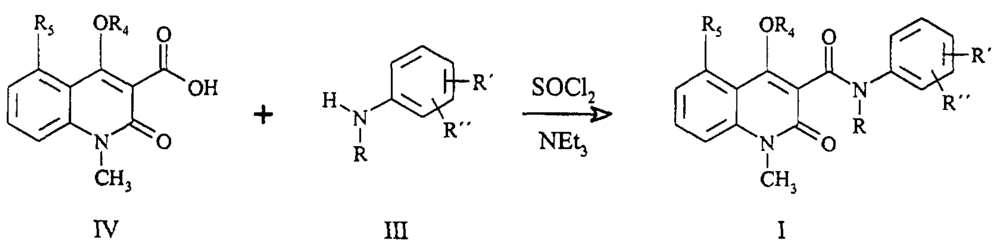


The compounds of formula (I) may also be prepared by reaction of a quinoline carboxylic acid of formula (IV) with an aniline of formula (III). Various coupling reagents known in the art may be used, e.g., carbodiimides known from literature in US Pat. No. 4,547,511. One suitable coupling method utilises thionyl chloride in the presence of triethylamine and a suitable solvent such as dichloromethane. This method may be used in instances when direct coupling between ester and aniline does not work, e.g., when the aniline contains electron withdrawing substituents. The quinoline carboxylic acids of formula (IV) may be obtained from the corresponding esters of formula (II) by acidic hydrolysis as described below.

*Example 1.*

1,2-Dihydro-4-hydroxy-5-thiomethyl-1-methyl-2-oxo-quinoline-3-carboxylic acid ethyl ester.

2,6-Difluorobenzonitrile (13.2 g, 0.10 mol) was slowly added to a solution of sodium sulphide nonahydrate (26.4 g, 0.11 mol) in 300 ml of N,N-dimethylformamide. After stirring the solution overnight, it was worked up with ether and 1 M hydrochloric acid. The ether phase was treated with 1 M sodium hydroxide and then the aqueous phase was acidified with hydrochloric acid followed by ether extraction. The ether phase was carefully dried and the solvent removed. The residue (12.5 g) was added in portions to a solution of sodium hydride (3.8 g, 0.12 mol) in 300 ml of cold N,N-dimethylformamide, followed by addition of methyl iodide (10 ml, 0.15 mol). The mixture was stirred for 6 hours at ambient temperature, poured onto ice-water and the resulting precipitate, 2-fluoro-6-methylthio-benzonitrile, filtered off and dried. The precipitate was warmed at 40°C in 200 ml of anhydrous methylamine in an autoclave for 2 days.

The excess methylamine was allowed to evaporate and the resulting yellow solid (16 g) was dissolved in 200 ml of dichloromethane together with 4-aminopyridine (0.1 g, 0.001 mol) and triethylamine (5.6 ml, 0.045 mol). To this chilled solution was slowly added ethyl malonyl chloride (15 g, 0.10 mol). The solution was stirred for 4 hours and worked up to give a yellowish syrup. The syrup was dissolved in 100 ml of anhydrous ethanol, and sodium methoxide (7.5 g, 0.14 mol) was added. After 1 hour, the solvent was removed and the residue worked up with dichloromethane and water. The quinoline derivative formed was suspended in 250 ml of chilled anhydrous tetrahydrofuran, and sodium hydride (5.3 g, 0.175 mol) was slowly added, followed by addition of methyl iodide (13 ml, 0.21 mol). The mixture was heated under reflux for 6 hours, quenched with water and worked up with diethyl ether. The solvents were removed and the residue (17.3 g) dissolved in a mixture of 100 ml of ethanol and 5 ml of conc. hydrochloric acid. The solution was warmed at 80°C for 4 hours, cooled and the precipitate was collected and purified by silica gel chromatography to give the title compound (4 g).
1H NMR ($CDCl_3$) δ 1.46 (3H, t), 2.48 (3H, s), 3.62 (3H, s), 4.48 (2H, q), 6.98 (1H, d), 7.05 (1H, d), 7.52 (1H, t), 15.5 (1H, s).

*Example 2.*

N-Ethyl-N-phenyl-1,2-dihydro-4-hydroxy-5-methylthio-1-methyl-2-oxo-quinoline-3-carboxamide.

N-Ethylaniline (3.0 g, 0.025 mol) was dissolved in 80 ml of toluene and about 30 ml of the solvent was distilled off in order to obtain a dry solution. 1,2-Dihydro-4-hydroxy-5-methylthio-1-methyl-2-oxo-quinoline-3-carboxylic acid ethyl ester (2.9 g, 0.01 mol) was added to the boiling solution. The ethanol formed during the reaction was distilled off together with some toluene for about 4 hours. The mixture was cooled to room temperature. The precipitate was collected, washed with cold toluene and hexane and dried to give the title compound (2.1 g), yield 57 %.
1H NMR ($CDCl_3$) δ 1.22 (3H, t), 2.47 (3H, s), 3.35 (3H, s), 3.96 (2H, q), 7.06 (1H, d), 7.09 (1H, d), 7.14-7.26 (5H, m), 7.45 (1H, t).
13C NMR ($CDCl_3$) δ 13.0 (CH3), 18.5 (CH3), 29.7 (CH3), 45.4 (CH2), 105.2 (C), 112.1 (CH), 113.1 (C), 121.8 (CH), 126.7 (CH), 126.9 (CH), 128.5 (CH), 131.4 (CH), 138.7 (C), 142.3 (C), 158.5 (C), 165.5 (C), 168.4 (C).
ESI MS/MS $[M+H]^+$ 369, fragments 248 and 122.

In essentially the same manner the following compounds were obtained from the corresponding starting materials:

N-allyl-N-phenyl-1,2-dihydro-4-hydroxy-5-methylthio-1-methyl-2-oxo-quinoline-3-carboxamide,

N-methyl-N-phenyl-1,2-dihydro-4-hydroxy-5-methylthio-1-methyl-2-oxo-quinoline-3-carboxamide (not included in the claims),
1H NMR ($CDCl_3$) δ 2.45 (3H, s), 3.38 (3H, s), 3.48 (3H, s), 7.04 (1H, d), 7.06 (1H, d), 7.11-7.25 (5H, m), 7.43 (1H, t).
13C NMR ($CDCl_3$) δ 18.3 (CH3), 29.8 (CH3), 38.4 (CH3), 104.6 (C), 112.0 (CH), 113.2 (C), 121.5 (CH), 125.7 (CH), 125.7 (CH), 126.8 (CH), 128.7 (CH), 128.7 (CH),131.5 (CH), 139.1 (C), 142.3 (C), 144.2 (C), 158.7 (C), 166.0 (C), 169.1 (C).
ESI MS/MS $[M+H]^+$ 355, fragments 248 and 108.

N-n-propyl-N-phenyl-1,2-dihydro-4-hydroxy-5-methylthio-1-methyl-2-oxo-quinoline-3-carboxamide,

N-methyl-N-(4-chloro-phenyl)-1,2-dihydro-4-hydroxy-5-methylthio-1-methyl-2-oxo-quinoline-3-carboxamide,

N-ethyl-N-(4-methoxy-phenyl)-1,2-dihydro-4-hydroxy-5-methylthio-1-methyl-2-oxo-quinoline-3-carboxamide,

N-methyl-N-(4-methoxy-phenyl)-1,2-dihydro-4-hydroxy-5-methylthio-1-methyl-2-oxo-quinoline-3-carboxamide,
1H NMR ($CDCl_3$) δ 2.43 (3H, s), 3.39 (3H, s), 3.40 (3H, s), 3. 72 (3H, s), 6.72 (2H, broad signal), 7.01-7.21 (4H, m), 7.44 (1H, t).
13C NMR ($CDCl_3$) δ 18.6 (CH3), 29.9 (CH3), 38.4 (CH3), 55.4 (CH3), 112.3 (CH), 113.3 (C), 113.9 (CH), 123 (CH), 127.1 (CH), 131.4 (CH), 137 (C), 138 (C), 142.3 (C), 158.2 (C), 158.7 (C), 169 (C).
ESI MS/MS $[M+H]^+$ 385, fragments 248 and 138.

*Example 3.*

N-Methyl-N-phenyl-1,2-dihydro-4-hydroxy-5-methylsulphinyl-1-methyl-2-oxo-quinoline-3-carboxamide (not included in the claims).

N-Methyl-N-phenyl-1,2-dihydro-4-hydroxy-5-methylthio-1-methyl-2-oxo-quinoline-3-carboxamide (0.14 g, 0.32 mmol) was dissolved in 5 ml of dichloromethane. The solution was cooled in an ice-bath and 70 % 3-chloroperoxybenzoic acid (83.3 mg, 0.34 mmol) dissolved in 2 ml of dichloromethane was slowly added. After stirring for 4 hours, the solution was triturated with 3 ml of heptane followed by recrystallization of the precipitate from ethyl acetate - pentane to give the title compound (25 mg).
1H NMR ($CDCl_3$) δ 2.79 (3H, s), 3.39 (3H, s), 3.50 (3H, s), 7.15-7.29 (5H, m), 7.35 (1H, d), 7.75 (1H, t), 8.06 (1H, d).
ESI MS/MS $[M+H]^+$ 371, fragment 264.
In essentially the same manner the following compound was obtained from the corresponding starting materials:

N-ethyl-N-phenyl-1,2-dihydro-4-hydroxy-5-methylsulphinyl-1-methyl-2-oxo-quinoline-3-carboxamide,

N-n-propyl-N-phenyl-1,2-dihydro-4-hydroxy-5-methylsulphinyl- 1-methyl-2-oxo-quinoline-3-carboxamide,

N-methyl-N-(4- methoxy-phenyl)-1,2-dihydro-4-hydroxy- 5-methylsulphinyl-1-methyl-2-oxo-quinoline-3-carboxamide,

N-methyl-N-(4- chloro-phenyl)-1,2-dihydro-4-hydroxy-5-methylsulphinyl-1-methyl-2-oxo-quinoline-3-carboxamide,

*Example 4.*

N-Methyl-N-(4-trifluoromethyl-phenyl)-1,2-dihydro-4-hydroxy-5-methylthio-1-methyl-2-oxo-quinoline-3-carboxamide (Method B).

To an ice-cold solution of 1,2-dihydro-4-hydroxy-5-methylthio-1-methyl-2-oxo-quinoline-3-carboxylic acid (8.5 g, 0.032 mol), triethylamine (15.5 ml, 0.11 mol) and 4-trifluoromethyl-N-methylaniline (6.1 g, 0.035 mol) in 150 ml of methylene chloride was added dropwise during 0.5 hours a solution of thionyl chloride (3.0 ml, 0.042 mol) in 10 ml of methylene chloride. The stirring was continued at 4°C for 4 hours. The solution was diluted with 10 ml of methylene chloride, washed with cold 1 M sulphuric acid and extracted with 1 M sodium hydroxide. The pH of the aqueous phase was adjusted to 8-8.5, it was clarified by filtration and then acidified with hydrochloric acid solution to pH 4. On standing a crystalline precipitate was formed which was filtered off, washed with water and dried to give the title compound (8.5 g) yield 72 %.
In essentially the same manner the following compounds were obtained from the corresponding starting materials:

N-ethyl-N-(4-trifluoromethyl-phenyl)-1,2-dihydro-4-hydroxy-5-methylthio-1-methyl-2-oxo-quinoline-3-carboxamide,

N-methyl-N-(4-trifluoromethoxy-phenyl)-1,2-dihydro-4-hydroxy-5-methylthio-1-methyl-2-oxo-quinoline-3-carboxamide,

N-ethyl-N-(2,4-difluoro-phenyl)-1,2-dihydro-4-hydroxy-5-methylthio-1-methyl-2-oxo-quinoline-3-carboxamide,

N-methyl-N-(2,4-difluoro-phenyl)-1,2-dihydro-4-hydroxy-5-methylthio-methyl-2-oxo-quinoline-3 -carboxamide,
1H NMR ($CDCl_3$) δ 2.46 (3H, s), 3.33 (3H, s), 3.37 (3H, s), 6.63 (1H, broad), 6.83 (1H, broad), 6.95-7.15 (3H, m broad), 7.45 (1H, t).
13C NMR ($CDCl_3$) δ 17.7 (CH3), 29.8 (CH3), 37.2 (CH3), 103.2 (C), 104.6+104.8+105.0 (CH), 110.5 (CH), 111.3 (CH), 112.7 (C), 120.1 (CH), 128.4 (C), 128.5 (CH), 131.9 (CH), 140.6 (C), 142.6 (C), 156.9+157.0+159.0+159.1 (C), 158.4 (C), 160.6+162.6 (C), 168.0 (C), 170.4 (C); some peaks are multiplets due to F-coupling.
ESI MS/MS $[M+H]^+$ 391, fragment 248.

N-methyl-N-(2,5-difluoro-phenyl)-1,2-dihydro-4-hydroxy-5-methylthio-1-methyl-2-oxo-quinoline-3-carboxamide

and

N-methyl-N-(4-trifluoromethyl-phenyl)-1,2-dihydro-4-hydroxy-5-methylsulphinyl-1-methyl-2-oxo-quinoline-3-carboxamide and

N-methyl-N-(2,4-difluoro-phenyl)-1,2-dihydro-4-hydroxy-5- methylsulphinyl-methyl-2-oxo-quinoline-3-carboxamide.

*Pharmacological methods*

Acute experimental autoimmune encephalomyelitis (aEAE).

SJL/N female mice, 8 weeks of age, were used for the experiments. Mouse spinal cord homogenate (MSCH) was obtained from 8 to 12 weeks-old C57B1/6 female mice. The tissue was homogenised on ice and diluted in cold PBS. Incomplete Freund's containing 1 mg/ml M. tuberculosis hominis H37Ra was emulsified with an equal volume of MSCH to give a final concentration of 10 mg/ml of MSCH. The inoculum volume of 0.1 ml was injected intradermally at the base of the tail. Pertussis toxin was injected i.p. at day 0 and 3 after immunization. Treatment was given per os daily either at day 3 to 12 post-immunization or days 3 to 7 and 10 to 12. Control animals received saline. The animals, eight per dose group, were scored for clinical signs of paralytic disease on a scale from 0 to 5 in the following way: 0, normal; 1, limp tail; 2, hind limb paresis; 3 hind limb paralysis and limp foreleg; 4, bilateral hind and fore limb paralysis; 5, death. Clinical scores were monitored at day 7 and daily from day 9 until the end of the experiment at day 14. Treatment effects were calculated as percent inhibition of clinical scores compared to saline treated controls.

Collagen induced arthritis.

DBA/1 male mice between 8 to 10 weeks of age were used for the experiments. On day 0 the mice were immunized intradermally at the base of the tail with bovine type II collagen (100 µg/mouse) in Freund's complete adjuvant. The treatment was given per os daily on days 3 to 7, 10 to 14, 17 to 21, 24 to 28 and 31 to 35. Fifteen days after immunization mice were inspected for signs of arthritis. The animals were inspected three times a week. Every second or third day individual paws of the arthritic animals were scored by a scale from 0-4 (0 = no arthritis, 1 = arthritis in one of the interphalangeal, metatarsophalangeal or intercarpal joints, 2 = two arthritic joints, 3 = three arthritic joints, 4 = as in 3 but with more severe redness and swelling of the paw). The score for each paw was added to give a maximal attainable score of 16 for each mouse.

Ovalbumin-induced lung inflammation.

C57B1/ 6 female mice between 10 to 14 weeks of age were used for the experiments, 10 mice /group. The mice were sensitized with ovalbumin (OA) in aluminium hydroxide in a volume of 0.2 ml inoculated ip. Treatment was given at day 0 to day 16. Control mice received saline. Fourteen days after the OA sensitization mice were exposed for 20 minutes to an aerosol of 1.5% w/v of OA in saline produced by a nebulizer. Vehicle-challenged control mice were exposed to saline. Seventy-two hours after OA/vehicle challenge, mice were anaesthetised and bronchoalveolar lavage was performed by instilling 0.5 ml ice-cold phosphate buffered saline (PBS) into the lungs twice. Total cell counts were determined and differential counts were made based on identification of eosinophils, monocytes/alveolar macrophages, lymphocytes and neutrophils. Eosinophil infiltration into the lung tissue was evaluated by histochemical methods on frozen lung sections using diaminobenzidine tetrahydrochloride (DAB).

*Teratogenic effects in the rat.*

The compounds were administrated subcutaneously to female rats during pregnancy, i.e., day 8 to 14 of pregnancy. The rats were caesarean sectioned and necropsied on day 20 after fertilisation. The foetuses were examined for external and internal abnormalities.

*Beagle Pain Syndrome (BPS).*

The compounds were administrated intravenously to beagle dogs. The dosage was given for five consecutive days. The dogs were evaluated for clinical and laboratory signs of the pain syndrome, e.g., fever, increased erythrocyte sedimentation rate (ESR), alkaline phosphate (AP), induction of acute phase proteins and vasculitis

Among preferred compounds are N-ethyl-N-phenyl-1,2-dihydro-4-hydroxy-5-methylthio-1-methyl-2-oxo-quinoline-3-carboxamide and N-methyl-N-(2,4-difluoro-phenyl)-1,2-dihydro-4-hydroxy-5-methylthio-1-methyl-2-oxo-quinoline-3-carboxamide hereinafter called Compound A and B, respectively. N-ethyl-N-phenyl-1,2-dihydro-4-hydroxy-6-methylthio-1-methyl-2-oxo-quinoline-3-carboxamide and N-methyl-N-(2,4-difluoro-phenyl)-1,2-dihydro-4-hydroxy-6-methylthio-1-methyl-2-oxo-quinoline-3-carboxamide are included as reference compounds hereinafter called Compound C and D, respectively:

*aEAE inhibition*

| Dose, mg/kg p.o. | % aEAE Inhibition | | | |
|---|---|---|---|---|
| | Compound A (invention) | Compound B (invention) | Compound C | Compound D |
| 0.04 | 28 | | 0 | |
| 0.2 | 49 | 67 | 23 | 0 |
| 1 | | 97 | | 66 |

*Teratogenic effects in the rat*

While in a dose of 6 mg/kg/day N-methyl-N-phenyl-1,2-dihydro-4-hydroxy-5-methylthio-1-methyl-2-oxo-quinoline-3-carboxamide was found to be teratogenic in the rat, Compound A and B were found to be non-teratogenic.

Effective quantities of the compounds of formula (I) are preferably administered to a patient in need of such treatment according to usual routes of administration and formulated in usual pharmaceutical compositions comprising an effective amount of the active ingredient and a suitable pharmaceutically acceptable carrier. Such compositions may take a variety of forms, e.g. solutions, suspensions, emulsions, tablets, capsules, and powders prepared for oral administration, aerosols for inhalation, sterile solutions for parental administration, suppositories for rectal administration or suitable topical formulations. Conventional procedures for the selection and preparation of suitable pharmaceutical formulations are described, for example, in "Pharmaceuticals - The Science of Dosage Form Design", M.B. Aulton, Churchill Livingstone, 1988.

A suitable daily dose for use in the treatment of MS is contemplated to vary between 0.0005 mg/kg to about 10 mg/kg body weight, in particular between 0.005 mg/kg to 1 mg/kg body weight, depending upon the specific condition to be treated, the age and weight of the specific patient, and the specific patient's response to the medication. The exact individual dosage, as well as the daily dosage, will be determined according to standard medical principles under the direction of a physician.

Various additives to enhance the stability or ease of administration of the drug are contemplated. The pharmaceutical composition may also contain additional therapeutically useful substances other than a compound of formula (I).

*References*

1. Talal, N.: Autoimmune diseases. In: Roitt, I.M. and Delves, P.J. (eds.) Encyclopedia of Immunology, pp. 195-198. Academic Press, 1992.
2. Prineas, J.W.: The neuropathology of multiple sclerosis. In: Koetsier, J.C. (ed.) Handbook of Clinical Neurology, pp. 213-257. Elsevier Science Publ., Amsterdam, 1985.
3. Karussis, D.M. Meiner, Z., Lehmann, D. et al. Treatment of secondary progressive multiple sclerosis with the immunomodulator Linomide. Neurology 47: 341-346, 1996.
4. Andersen, O., Lycke, J., Tollesson, P.O. et al. Linomide reduces the rate of active lesions in relapsing-remitting multiple sclerosis. Neurology 47: 895-900, 1996.
5. Kelly, D.F., Grimsell, C.S.G. and Kenyon, C.J. Polyarteritis in the dog: A case report. Vet Record 92: 363-366, 1973.
6. Harcourt, R.A. Polyarterites in a colony of beagles. Vet Record 102: 519-522, 1978.

## Claims

1. Compounds of general formula (I)

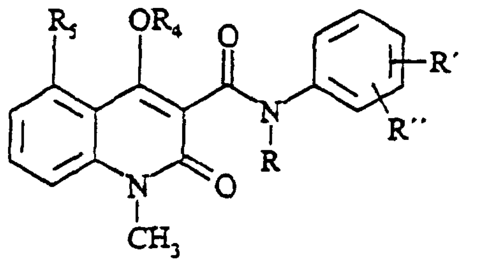

(I)

wherein
R is selected from Me, Et, n-Pr, iso-Pr, n-Bu, iso-Bu, sec.-Bu and allyl;
R' is selected from hydrogen, Me, MeO, fluoro, chloro, bromo, $CF_3$, and $OCH_xF_y$;
R" is selected from hydrogen and fluoro, with the proviso that R" is fluoro when R' is fluoro and further provided that when R' and R" are both hydrogen R is not Me;
$R_4$ is selected from hydrogen and pharmaceutically acceptable inorganic and organic cations;
$R_5$ is selected from MeS, EtS, n-PrS, iso-PrS, MeSO, EtSO, $MeSO_2$ and $EtSO_2$;
wherein

$$x = 0 - 2,$$

$$y = 1 - 3$$

with the proviso that

$$x + y = 3;$$

and
Me is methyl, Et is ethyl, Pr is propyl and Bu is butyl;
and any tautomer, optical isomer and racemate thereof.

2. Compounds according to claim 1 wherein the pharmaceutically acceptable inorganic cations are derived from sodium, potassium and calcium, and the organic cations are derived from monoethanolamine, diethanolamine, dimethylaminoethanol, morpholine.

3. Compounds according to claims 1 and 2 wherein $R_5$ is selected from MeS, and EtS.

4. Compounds according to claims 1 and 2 wherein R is methyl when at least one of R' and R" is not hydrogen.

5. Compounds according to claims 1 and 2 wherein R is ethyl or propyl and R' and R" are both hydrogen.

6. The compound according to claims 1, 2, 3 and 5, N-n-propyl-N-phenyl-1,2-dihydro-4-hydroxy-5-methylthio-1-methyl-2-oxo-quinoline-3-carboxamide.

7. The compound according to claims 1, 2, 3 and 4, N-methyl-N-(4-trifluoromethyl-phenyl)-1,2-dihydro-4-hydroxy-5-methylthio-1-methyl-2-oxo-quinoline-3-carboxamide.

8. The compound according to claims 1, 2, 3 and 4, N-methyl-N-(2,4-difluoro-phenyl)-1,2-dihydro-4-hydroxy-5-methylthio-1-methyl-2-oxo-quinoline-3-carboxamide.

**9.** The compound according to claims 1, 2, 3 and 4, N-methyl-N-(2,5-difluoro-phenyl)-1,2-dihydro-4-hydroxy-5-methylthio-1-methyl-2-oxo-quinoline-3-carboxamide.

**10.** The compound according to claims 1, 2, and 5, N-n-propyl-N-phenyl-1,2-dihydro-4-hydroxy-5-methylsulphinyl-1-methyl-2-oxo-quinoline-3-carboxamide.

**11.** The compounds according to any of the preceding claims for use as therapeuticum.

**12.** Pharmaceutical compositions containing as active ingredient a compound having the general formula (I) as defined in claim 1 together with a pharmaceutically carrier.

**13.** Pharmaceutical compositions according to claim 12 containing other pharmacologically active substances.

**14.** Pharmaceutical compositions according to claims 12 and 13 for use as therapeuticum in a daily dose of the active substance of 0.0005 mg/kg to about 10 mg/kg body weight, in particular 0.005 to 1 mg/kg body weight

**15.** A process for the manufacturing of a compound of the general formula (I) wherein R, R', R", $R_4$ and $R_5$ are as defined in claim 1, by

(A) reacting an ester derivative of quinoline carboxylic acid of formula (II)

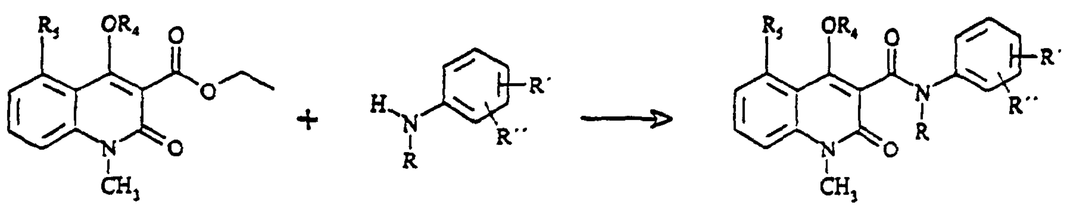

II III I

with an aniline of formula (III), in a suitable solvent such as toluene, xylene, or
(B) reacting an quinoline carboxylic acid of the general formula (IV) with an aniline of the general formula (III),

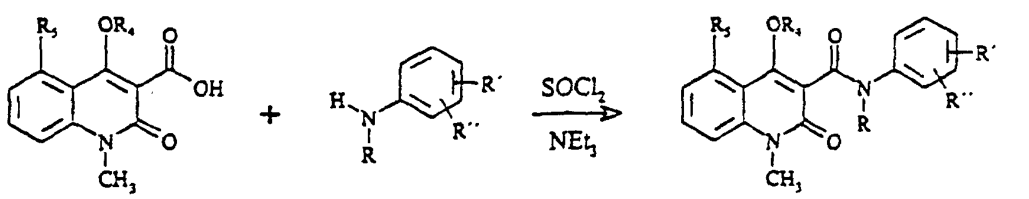


IV III I

using a suitable coupling reagent, preferably a carbodiimide or thionyl chloride in the presence of triethylamine and a suitable solvent such as dichloromethane.

**Patentansprüche**

**1.** Verbindungen der allgemeinen Formel (I)

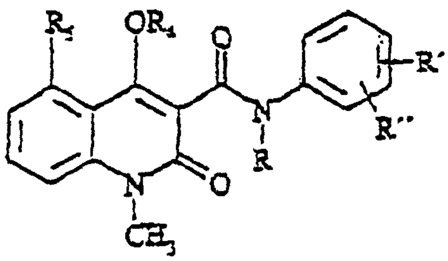

(I)

wobei
R aus Me, Et, n-Pr, iso-Pr, n-Bu, iso-Bu, sek.-Bu und Allyl ausgewählt ist;
R' aus Wasserstoff, Me, MeO, Fluor, Chlor, Brom, $CF_3$ und $OCH_xF_y$ ausgewählt ist;
R" aus Wasserstoff und Fluor ausgewählt ist, mit der Maßgabe, dass R" Fluor ist, wenn R' Fluor ist, und mit der weiteren Maßgabe, dass wenn R' und R" beide Wasserstoff sind, R nicht Me ist;
$R_4$ aus Wasserstoff und pharmazeutisch verträglichen anorganischen und organischen Kationen ausgewählt ist;
$R_5$ aus MeS, EtS, n-PrS, iso-PrS, MeSO, EtSO, $MeSO_2$ und $EtSO_2$ ausgewählt ist;
wobei

$$x = 0\text{-}2$$

$$y = 1\text{-}3$$

mit der Maßgabe, dass

$$x+y = 3;$$

und
Me Methyl, Et Ethyl, Pr Propyl und Bu Butyl ist;
und jegliches Tautomer, optisches Isomer und Racemat davon.

**2.** Verbindungen gemäß Anspruch 1, wobei die pharmazeutisch verträglichen anorganischen Kationen von Natrium, Kalium und Calcium abgeleitet sind und die organischen Kationen von Monoethanolamin, Diethanolamin, Dimethylaminoethanole und Morpholin abgeleitet sind.

**3.** Verbindungen gemäß den Ansprüchen 1 und 2, wobei $R_5$ aus MeS und EtS ausgewählt ist.

**4.** Verbindungen gemäß den Ansprüchen 1 und 2, wobei R Methyl ist, wenn mindestens einer von der R' und R" nicht Wasserstoff ist.

**5.** Verbindungen gemäß den Ansprüchen 1 und 2, wobei R Ethyl oder Propyl ist und R' und R" beide Wasserstoff sind.

**6.** Verbindung gemäß den Ansprüchen 1, 2, 3 und 5, nämlich N-n-Propyl-N-phenyl-3,2-dihydro-4-hydroxy-5-methylthio-1-methyl-2-oxo-chinolin-3-carboxamid.

**7.** Verbindung gemäß den Ansprüchen 1, 2, 3 und 4, nämlich N-Methyl-N-(4-trifluormethyl-phenyl)-1,2-dihydro-4-hydroxy-5-methylthio-1-methyl-2-oxo-chinolin-3-carboxamid.

**8.** Verbindung gemäß den Ansprüchen 1, 2, 3 und 4, nämlich N-Methyl-N-(2,4-difluorphenyl)-1,2-dihydro-4-hydroxy-5-methylthio-1-methyl-2-oxo-chinolin-3-carboxamid.

**9.** Verbindung gemäß den Ansprüchen 1, 2, 3 und 4, nämlich N-Methyl-N-(2,5-difluorphenyl)-1,2-dihydro-4-hydroxy-5-methylthio-1-methyl-2-oxo-chinolin-3-carboxamid.

**10.** Verbindung gemäß den Ansprüchen 1, 2 und 5, nämlich N-n-Propyl-N-phenyl-1,2-dihydro-4-hydroxy-5-methylsulphenyl-1-methyl-2-oxo-chinolin-3-carboxamid.

**11.** Verbindungen gemäß einem der vorangegangenen Ansprüche zur Verwendung als Therapeutikum.

**12.** Arzneimittel, die als Wirkstoff eine Verbindung der allgemeinen Formel (I), wie im Anspruch 1 definiert, zusammen mit einem pharmazeutisch verträglichen Träger enthalten.

**13.** Arzneimittel gemäß Anspruch 12, die andere pharmakologische Wirkstoffe enthalten.

**14.** Arzneimittel gemäß den Ansprüchen 12 und 13 zur Verwendung als Therapeutikum in einer Tagesdosis des Wirkstoffs von 0,0005 mg/kg bis etwa 10 mg/kg Körpergewicht, insbesondere 0,005 bis 1 mg/kg Körpergewicht.

**15.** Verfahren zur Herstellung einer Verbindung der allgemeinen Formel (I), wobei R, R', R'', $R_4$, und $R_5$ wie in Anspruch 1 definiert sind, durch

(A) Umsetzen eines Esterderivates der Chinolincarbonsäure der Formel (II)

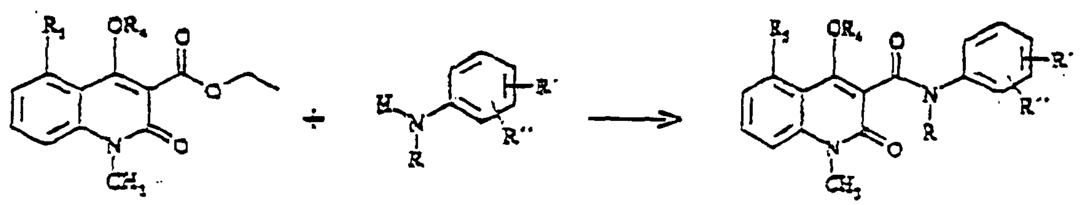

II III I

mit einem Anilin der Formel (III) in einem geeigneten Lösemittel wie etwa Toluol, Xylol oder
(B) Umsetzen einer Chinolincarbonsäure der allgemeinen Formel (IV) mit einem Anilin der allgemeinen Formel (III)

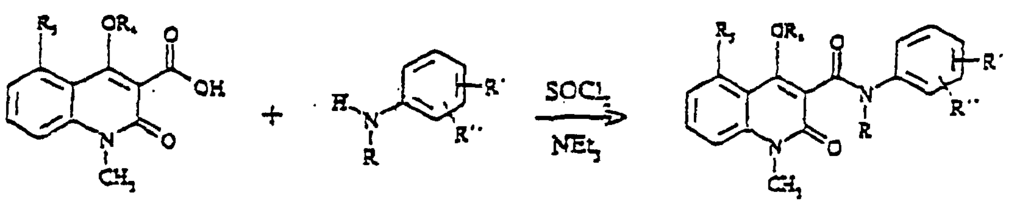


IV III I

unter Verwendung eines geeigneten Kupplungsreagenz, vorzugsweise eines Carbodiimids oder Thionylchlorids, in Anwesenheit von Triethylamin und einem geeigneten Lösemittel wie etwa Dichlormethan.

## Revendications

**1.** Composés de formule générale (I)

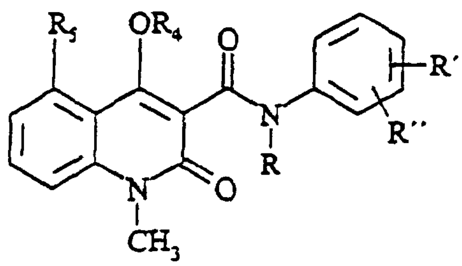

(I)

dans laquelle

R est choisi parmi les groupes Me, Et, n-Pr, iso-Pr, n-Bu, iso-Bu, sec.-Bu et allyle ;
R' est choisi parmi un atome d'hydrogène, les groupes Me, MeO, fluoro, chloro, bromo, $CF_3$ et $OCH_xF_y$ ;
R" est choisi parmi un atome d'hydrogène et un groupe fluoro, à condition que R" soit un groupe fluoro lorsque R' est un groupe fluoro et à condition, en outre, que lorsque R' et R" sont tous les deux des atomes d'hydrogène, R ne soit pas un groupe Me ;
$R_4$ est choisi parmi un atome d'hydrogène et les cations organiques et inorganiques pharmaceutiquement acceptables ;
$R_5$ est choisi parmi les groupes MeS, EtS, n-PrS, iso-PrS, MeSO, EtSO, $MeSO_2$ et $EtSO_2$ ;
où

$$x = 0 \text{ à } 2,$$

$$y = 1 \text{ à } 3,$$

à condition que

$$x + y = 3 ;$$

et
Me signifie méthyle, Et signifie éthyle, Pr signifie propyle et Bu signifie butyle;

et l'un quelconque de leurs tautomères, isomères optiques et racémates.

**2.** Composés selon la revendication 1, dans lesquels les cations inorganiques pharmaceutiquement acceptables sont dérivés du sodium, du potassium et du calcium, et les cations organiques sont dérivés de la monoéthanolamine, de la diéthanolamine, du diméthylaminoéthanol et de la morpholine.

**3.** Composés selon les revendications 1 et 2, dans lesquels $R_5$ est choisi parmi les groupes MeS et EtS.

**4.** Composés selon les revendications 1 et 2, dans lesquels R est un groupe méthyle lorsqu'au moins un élément parmi R' et R" n'est pas un atome d'hydrogène.

**5.** Composés selon les revendications 1 et 2, dans lesquels R est un groupe éthyle ou propyle et R' et R" sont tous deux des atomes d'hydrogène.

**6.** Composé selon les revendications 1, 2, 3 et 5, le N-n-propyl-N-phényl-1,2-dihydro-4-hydroxy-5-méthylthio-l-méthyl-2-oxo-quinoléine-3-carboxamide.

**7.** Composé selon les revendications 1, 2, 3 et 4, le N-méthyl-N-(4-trifluorométhyl-phényl)-1,2-dihydro-4-hydroxy-5-méthylthio-1-méthyl-2-oxo-quinoléine-3-carboxamide.

**8.** Composé selon les revendications 1, 2, 3 et 4, le N-méthyl-N-(2,4-difluoro-phényl)-1,2-dihydro-4-hydroxy-5-méthylthio-1-méthyl-2-oxo-quinoléine-3-carboxamide.

**9.** Composé selon les revendications 1, 2, 3 et 4, le N-méthyl-N-(2,5-difluoro-phényl)-1,2-dihydro-4-hydroxy-5-méthylthio-1-méthyl-2-oxo-quinoléine-3-carboxamide.

**10.** Composé selon les revendications 1, 2 et 5, le N-n-propyl-N-phényl-1,2-dihydro-4-hydroxy-5-méthylsulfinyl-1-méthyl-2-oxo-quinoléine-3-carboxamide.

**11.** Composés selon l'une quelconque des revendications précédentes pour une utilisation en tant qu'agents thérapeutiques.

**12.** Compositions pharmaceutiques contenant, en tant que principe actif, un composé de formule générale (I) telle que définie dans la revendication 1, conjointement avec un véhicule pharmaceutiquement acceptable.

**13.** Compositions pharmaceutiques selon la revendication 12 contenant d'autres substances pharmacologiquement actives.

**14.** Compositions pharmaceutiques selon les revendications 12 et 13 pour une utilisation en tant qu'agents thérapeutiques, en dose quotidienne du principe actif de 0,0005 mg/kg à environ 10 mg/kg de poids corporel, en particulier de 0,005 à 1 mg/kg de poids corporel.

**15.** Procédé de fabrication d'un composé de formule générale (I) dans laquelle R, R', R", $R_4$ et $R_5$ sont tels que définis dans la revendication 1, consistant à

(A) faire réagir un dérivé ester d'un acide quinoléine carboxylique de formule (II)

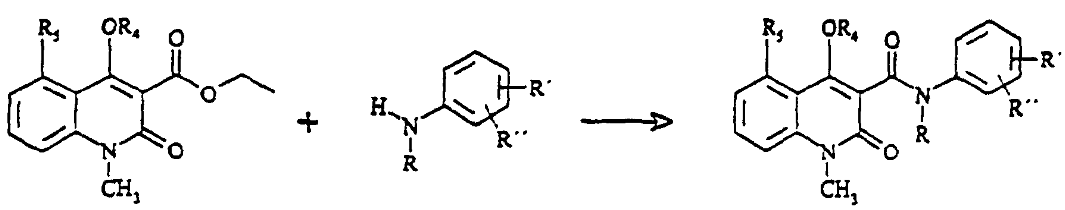

II III I

avec une aniline de formule (III), dans un solvant approprié tel que le toluène, le xylène, ou

(B) faire réagir un acide quinoléine carboxylique de formule générale (IV) avec une aniline de formule générale (III),

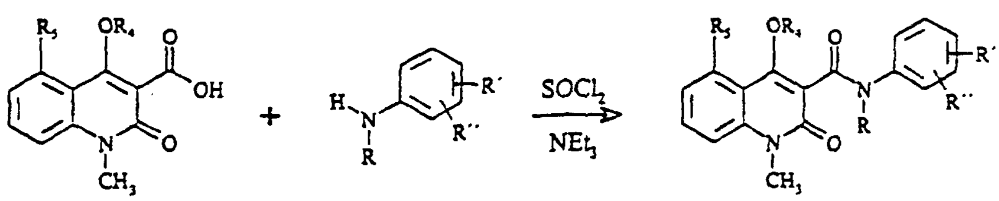


IV III I

en utilisant un réactif de couplage approprié, de préférence un carbodiimide ou un chlorure de thionyle en présence de triéthylamine, et un solvant approprié tel que le dichlorométhane.